Europäisches Patentamt
European Patent Office
Office européen des brevets

(19)

(11) Veröffentlichungsnummer: **0 373 497 A2**

(12)

# EUROPÄISCHE PATENTANMELDUNG

(21) Anmeldenummer: **89122519.5**

(51) Int. Cl.5: **C07K 5/06, A61K 37/64**

(22) Anmeldetag: **06.12.89**

Patentansprüche für folgende Vertragsstaaten: ES + GR

(30) Priorität: **10.12.88 DE 3841732**

(43) Veröffentlichungstag der Anmeldung: **20.06.90 Patentblatt 90/25**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Nickel, Wolf-Ulrich, Dr.**
**Robert-Stolz-Strasse 120**
**D-6232 Bad Soden am Taunus(DE)**
Erfinder: **Urbach, Hansjörg, Dr.**
**Le Lavandoustrasse 41**
**D-6242 Kronberg/Taunus(DE)**
Erfinder: **Ruppert, Dieter, Dr.**
**Schreyerstrasse 30**
**D-6242 Kronberg/Taunus(DE)**
Erfinder: **Schölkens, Bernward, Dr.**
**Hölderlinstrasse 62**
**D-6233 Kelkheim((Taunus)(DE)**

(54) **Dipeptid-Derivate mit Enzym-inhibitorischer Wirkung.**

(57) Verbindungen der Formel I,

$$R^1-\overset{\overset{\large O}{\|}}{C}-A-B-NH-\overset{\overset{\large R^2}{|}}{CH}-\overset{\overset{\large OH}{|}}{CH}-\overset{\overset{\large R^3}{|}}{CH}-R^4$$

in welcher $R^1$ für ein (subst.) Heterocyclyl-alkyl, -alkoxy, -cycloalkyl, -cycloalkoxy und die entsprechenden Heterocyclylmercapto-Verbindungen steht, A und B unabhängig voneinander eine Aminosäure bedeuten, $R^2$ Wasserstoff, Alkyl, Cycloalkyl, Cycloalkyl-alkyl, Aryl oder Aryl-alkyl ist, $R^3$ für Wasserstoff, Alkyl, Aryl, Aryl-alkyl oder Hydroxy steht und $R^4$ einen Rest $(CH_2)_{0-4}CHR^5$-D, mit $R^5$ = Wasserstoff, Alkyl, Alkoxy, Alkylthio, Alkylamino, Hydroxy, Azido oder Halogen und D = (subst.) Heterocyclyl, bedeutet, weisen enzymhemmende Eigenschaften auf. Es werden Verfahren zur Herstellung von Verbindungen der Formel I sowie ihre Verwendung bei der Behandlung des Bluthochdrucks und viraler Erkrankungen beschrieben.

EP 0 373 497 A2

# Dipeptid-Derivate mit Enzym-inhibitorischer Wirkung

Die Erfindung betrifft Dipeptid-Derivate, die die Wirkung des natürlichen Enzyms Renin und viraler Aspartylproteasen hemmen, sowie Verfahren zu ihrer Herstellung und ihre Verwendung.

Aus den EP-A-172 346, EP-A-172 347, EP-A-189 203, EP-A-229 667, EP-A-230 266, EP-A-255 082, EP-A-273 893 und EP-A-274 259 sind Dipeptid-Derivate und deren Verwendung als Renin-Inhibitoren bekannt.

Es wurden nun neue Dipeptid-Derivate gefunden, die in vitro und in vivo hochwirksam das Enzym Renin hemmen.

Die Erfindung betrifft Verbindungen der Formel I

$$R^1-\overset{O}{\overset{\|}{C}}-A-B-NH-\overset{R^2}{\overset{|}{C}H}-\overset{OH}{\overset{|}{C}H}-\overset{R^3}{\overset{|}{C}H}-R^4 \qquad (I),$$

in welcher
$R^1$ Het($C_1$-$C_6$)-alkyl,
Het-($C_1$-$C_6$)-alkoxy,
Het-($C_3$-$C_8$)-cycloalkyl,
Het-($C_3$-$C_8$)-cycloalkyl-($C_1$-$C_4$)-alkyl,
Het-($C_3$-$C_8$)-cycloalkoxy,
Het-($C_3$-$C_8$)-cycloalkoxy-($C_1$-$C_4$)-alkyl,
Het-mercapto-($C_1$-$C_6$)-alkyl,
Het-mercapto-($C_3$-$C_8$)-cycloalkyl,
Het-mercapto-($C_3$-$C_8$)-cycloalkyl-($C_1$-$C_4$)-alkyl,
Het-mercapto-($C_1$-$C_6$)-alkoxy,
Het-mercapto-($C_3$-$C_8$)-cycloalkoxy bedeutet,
wobei Het für einen 5- bis 7-gliedrigen monocyclischen oder 8- bis 10-gliedrigen bicyclischen heterocyclischen Ring steht, der benzannelliert, aromatisch, teilhydriert oder vollständig hydriert sein kann, der als Heteroelemente einen, zwei oder drei, gleiche oder verschiedene Reste aus der Gruppe N, O, S, NO, SO, $SO_2$ enthalten kann und der durch einen oder zwei, gleiche oder verschiedene Reste aus der Gruppe ($C_1$-$C_6$)-Alkyl, ($C_1$-$C_6$)-Alkoxy, ($C_1$-$C_6$)-Alkoxycarbonyl, Hydroxy, Halogen, Amino, Mono- oder Di-($C_1$-$C_6$)-alkylamino, Oxido substituiert sein kann;
$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl oder ($C_6$-$C_{14}$)-Aryl-($C_1$-$C_4$)-alkyl bedeutet;
$R^3$ Wasserstoff, ($C_1$-$C_{10}$)-Alkyl, ($C_6$-$C_{14}$)-Aryl, ($C_6$-$C_{14}$)-Aryl-($C_1$-$C_4$)-alkyl oder Hydroxy bedeutet;
$R^4$ einen Rest der Formel II bedeutet,
$(CH_2)_m$-$CHR^5$-D (II)
wobei
$R^5$ für Wasserstoff, ($C_1$-$C_7$)-Alkyl, ($C_1$-$C_5$)-Alkoxy, ($C_1$-$C_5$)-Alkylthio, ($C_1$-$C_5$)-Alkylamino, Hydroxy, Azido, Fluor, Chlor, Brom, Jod steht,
D einen Rest Het bedeutet, wobei
Het einen 5- bis 7-gliedrigen heterocyclischen Ring steht, der benzannelliert, aromatisch, teilhydriert oder vollständig hydriert sein kann, der als Heteroatome einen oder zwei gleiche oder verschiedene Reste aus der Gruppe N, O, S, NO, SO, $SO_2$ enthalten kann und der durch einen oder zwei gleiche oder verschiedene Reste aus der Gruppe ($C_1$-$C_4$)-Alkyl, ($C_1$-$C_4$)-Alkoxy, Hydroxy, Halogen, Amino, Mono- oder Di-($C_1$-$C_4$)-alkylamino, $CF_3$ substituiert sein kann, und m 0, 1, 2, 3 oder 4 bedeutet;
A und B unabhängig voneinander einen N-terminal mit $R^1$ bzw. A und C-terminal mit B bzw. NH-$CHR^2$-CHOH-$CHR^3$-$R^4$ verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, 2-Amino-4-(3-thienyl)-buttersäure, 3-(2-Thienyl)-serin, (Z)-Dehydrophenylalanin, (E)-Dehydrophenylalanin, 1,3-Dioxolan-2-yl-alanin, N-Pyrrolylalanin und 1-, 3- oder 4-Pyrazolylalanin bedeuten, sowie deren physiologisch verträgliche Salze.

Die Chiralitätszentren in den Verbindungen der Formel I können die R-, S- oder R-S-Konfiguration aufweisen.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

($C_6$-$C_{14}$)-Aryl ist beispielsweise Phenyl, Naphthyl, Biphenylyl oder Fluorenyl; bevorzugt ist Phenyl. Entsprechendes gilt für davon abgeleitete Reste, wie z.B. Aryloxy, Aroyl, Aralkyl und Aralkyloxy. Unter Aralkyl versteht man einen mit ($C_1$-$C_6$)-Alkyl verknüpften unsubstituierten oder substituierten ($C_6$-$C_{14}$)-Aryl-Rest, wie z.B. Benzyl, α- und β-Naphthylmethyl, Halobenzyl und Alkoxybenzyl, wobei Aralkyl jedoch nicht auf die genannten Reste beschränkt wäre.

Ein Rest Het im Sinne vorstehender Definition ist beispielsweise Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benzannelliertes, cyclopenta-, cyclohexa- oder cyclohepta-annelliertes Derivat dieser Reste. Dieser Heterocyclus kann an einem Stickstoffatom durch Oxido, ($C_1$-$C_6$)-Alkyl, z.B. Methyl oder Ethyl, Phenyl oder Phenyl-($C_1$-$C_4$)-alkyl, z.B. Benzyl, und/oder an einem oder mehreren Kohlenstoffatomen durch ($C_1$-$C_4$)-Alkyl, z.B. Methyl, Phenyl, Phenyl-($C_1$-$C_4$)-alkyl, z.B. Benzyl, Halogen, z.B. Chlor, Hydroxy, ($C_1$-$C_4$)-Alkoxy, z.B. Methoxy, Phenyl-($C_1$-$C_4$)-alkoxy, z.B. Benzyloxy, oder Oxo substituiert und teilweise gesättigt sein und ist beispielsweise 2- oder 3-Pyrrolyl, Phenyl-pyrrolyl, z.B. 4-oder 5-Phenyl-2-pyrrolyl, 2-Furyl, 2-Thienyl, 4-Imidazolyl, Methyl-imidazolyl, z.B. 1-Methyl-2-, 4- oder 5-imidazolyl, 1,3-Thiazol-2-yl, 2-, 3- oder 4-Pyridyl, 1-Oxido-2-, 3-oder 4-pyridino, 2-Pyrazinyl, 2-, 4- oder 5-Pyrimidinyl, 2-, 3- oder 5-Indolyl, substituiertes 2-Indolyl, z.B. 1-Methyl-, 5-Methyl-, 5-Methoxy, 5-Benzyloxy-, 5-Chlor-oder 4,5-Dimethyl-2-indolyl, 1-Benzyl-2- oder 3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl oder β-Carbolin-3-yl.

Teilhydrierte oder vollständig hydrierte heterocyclische Ringe sind beispielsweise Dihydropyridinyl, Pyrrolidinyl, z.B. 2-, 3- oder 4-N-Methylpyrrolidinyl, Piperidinyl, Piperazinyl, Morpholino, Thiomorpholino, Tetrahydrothiophenyl.

Halogen steht für Fluor, Chlor, Brom oder Jod, insbesondere für Fluor oder Chlor.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Bevorzugt sind Verbindung der Formel I, in welcher

$R^1$ wie auf Seite 1 definiert ist;

$R^2$ Isobutyl, Benzyl oder Cyclohexylmethyl bedeutet;

$R^3$ Wasserstoff, ($C_1$-$C_5$)-Alkyl, ($C_6$-$C_{10}$)-Aryl, ($C_6$-$C_{10}$)-Aryl-($C_1$-$C_4$)-alkyl oder Hydroxy bedeutet;

$R^4$ einen Rest der Formel II bedeutet, worin

$R^5$ für Wasserstoff, ($C_1$-$C_4$)-Alkyl, ($C_1$-$C_4$)-Alkoxy, ($C_1$-$C_4$)-Alkylthio, ($C_1$-$C_4$)-Alkylamino, Hydroxy, Azido, Fluor, Chlor, Brom oder Jod steht,

D wie der Rest Het auf Seite 2 definiert ist, und

m 0, 1 oder 2 bedeutet; und

A und B unabhängig voneinander einen bivalenten Rest aus der Reihe

Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, Norleucin, Cystein, 5-Methyl-Cystein, 1,2,3,4-Tetrahydroisochinolin-3-carbonsäure, Homophenylalanin, 2-Amino-4-(2-thienyl)-buttersäure, 2-Amino-4-(3-thienyl)-buttersäure, 3-(2- Thienyl)-serin, (Z)-Dehydrophenylalanin, (E)-Dehydrophenylalanin, 1,3-Dioxolan-2-yl-alanin, N-Pyrrolylalanin und 1-, 3- oder 4-Pyrazolylalanin, bedeuten, sowie deren physiologisch verträgliche Salze.

Insbesondere bevorzugt sind Verbindungen der Formel I, in welcher

$R^1$ Het-($C_1$-$C_4$)-alkyl,
Het-($C_1$-$C_4$)-alkoxy,
Het-($C_5$-$C_6$)-cycloalkyl,
Het-($C_5$-$C_6$)-cycloalkoxy,
Het-mercapto-($C_1$-$C_3$)-alkyl,
Het-mercapto-($C_5$-$C_6$)-cycloalkyl,
Het-mercapto-($C_1$-$C_3$)- alkoxy,
Het-mercapto-($C_5$-$C_6$)-cycloalkoxy bedeutet,
wobei Het für einen 5- bis 6-gliedrigen heterocyclischen Ring steht, wie z.B. Furanyl, Thienyl, Pyrrolyl, Imidazolyl, Isoxazolyl, Thiazolyl, Pyrazolyl, Triazolyl, Pyridyl, Pyrimidyl, Pyrazinyl, Pyridazinyl, Triazinyl, Pyrrolidyl, Piperidyl, Piperazinyl, Morpholino, Thiomorpholino, Tetrahydrofuranyl, Tetrahydropyranyl, Tetrahydrothienyl, und durch einen oder zwei gleiche oder verschiedene Reste aus der Gruppe ($C_1$-$C_4$)-Alkyl, ($C_1$-$C_4$)-Alkoxy, ($C_1$-$C_4$)-Alkoxycarbonyl, Hydroxy, Amino, Mono- oder Di-($C_1$-$C_4$)-Alkylamino und Oxido substituiert sein kann;
$R^2$ Isobutyl, Benzyl oder Cyclohexylmethyl bedeutet;
$R^3$ Wasserstoff oder Hydroxy bedeutet;
$R^4$ einen Rest der Formel II bedeutet, in welcher
$R^5$ für Wasserstoff oder Fluor steht,
D für einen 2-, 3- oder 4-Pyridinrest, einen 2-, 4- oder 5-Imidazolrest oder einen 2-Oxazolinrest steht, wobei die genannten Heterocyclen jeweils durch einen oder zwei Reste aus der Gruppe Methyl, Methoxy, Fluor, Chlor, Brom und $CF_3$ substituiert sein können, und
m 0, 1 oder 2 bedeutet; und
A und B unabhängig voneinander einen bivalenten Rest aus der Reihe
Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, Norleucin, 1,2,3,4-Tetrahydroisochinolin-3-carbonsäure, Homophenylalanin, 2-Amino-4-(2-thienyl)-buttersäure und 1-, 3- und 4-Pyrazolylalanin bedeuten,
sowie deren physiologisch verträgliche Salze.

Die Erfindung betrifft weiterhin ein Verfahren zur Herstellung von Verbindungen der Formel I, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel I mit einer endständigen Carboxylgruppe besitzen die nachstehenden Formeln IIIa, IIIb, IIIc:

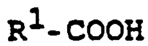
IIIa

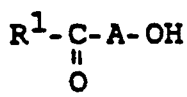
IIIb

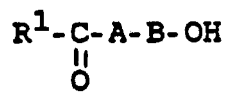
IIIc

Fragmente einer Verbindung der Formel I mit einer endständigen Aminogruppe besitzen die nachstehenden Formeln IVa, IVb, IVc:

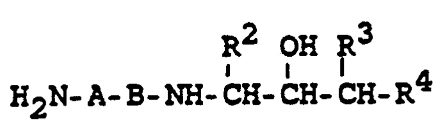
IVa

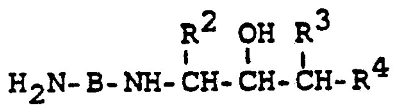

IVb

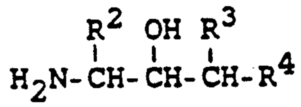

IVc

Methoden, die zur Herstellung einer Amidbindung geeignet sind, werden z.B. in Houben-Weyl, Methoden der organischen Chemie, Band 15/2; Bodanszky et al., Peptide synthesis, 2nd ed. (Wiley & Sons, New York 1976) oder Gross, Meienhofer, The Peptides. Analysis, synthesis, biology (Academic Press, New York 1979) beschrieben. Vorzugsweise werden die folgenden Methoden herangezogen:
Aktivestermethode mit N-Hydroxy-succinimid oder 1-Hydroxybenzotrizol als Esterkomponente, Kupplung mit einem Carbodiimid wie Dicyclohexylcarbodiimid oder mit Propanphosphonsäureanhydrid und die Gemischt-Anhydrid-Methode mit Pivaloylchlorid.

Die Herstellung der als Ausgangsverbindungen verwendeten optisch aktiven Amine der Formel IVc

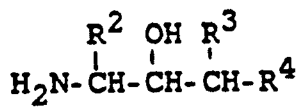

IVc

worin $R^2$, $R^3$ und $R^4$ wie oben definiert sind, erfolgt ausgehend von optisch aktiven α-Aminosäuren, wobei deren Asymmetriezentrum erhalten bleibt. Hierzu wird in bekannter Weise ein N-geschützter Aminosäurealdehyd hergestellt, welcher in einer Aldol-analogen Addition an einen entsprechenden Heteroarylalkyl-Baustein gekuppelt wird und nach Abspaltung der N-Schutzgruppe Aminoalkohole der Formel IVc ergibt. Man erhält Diastereomerengemische bezüglich des OH-tragenden Zentrums, die in an sich bekannter Weise, beispielsweise durch fraktionierte Kristallisation oder durch Chromatographie getrennt werden. Die Überprüfung der Diastereomerenreinheit erfolgt mittels HPLC, die Enantiomerenreinheit kann in bekannter Weise durch Überführung in Mosher-Derivate überprüft werden (H.S. Mosher et. al., J. org. Chem. 34, 2543 (1969)).

Die Herstellung N-geschützter Aminosäurealdehyde erfolgt nach B. Castro et al. (Synthesis 1983, 676).

Die Aldol-analoge Addition an N-geschützte Aminosäurealdehyde (bevorzugterweise N-tert.-Butoxycarbonyl-und Benzyloxycarbonyl-Schutzgruppen) erfolgt in einem gegenüber Basen inerten Lösungsmittel, wie Ether, THF, Toluol, DMF, DMSO oder Dimethoxyethan.

Als Basen zur Deprotonierung der Heteroarylalkyl-Komponente können Alkalimetallalkoholate, wie Kalium-O-tert.-butylat, Natriummethylat, Alkalimetallhydride, wie Natrium- oder Kaliumhydrid, metallorganische Basen, wie n-Butyllithium, s-Butyllithium, Methyllithium oder Phenyllithium, Natriumamid sowie Alkalimetallsalze von organischen Stickstoffbasen, wie Lithiumdiisopropylamid verwendet werden.

Die zur Herstellung von Verbindungen der Formel I erforderlichen Vor- und Nachoperationen wie Einführung und Abspaltung von Schutzgruppen sind literaturbekannt und sind z.B. in T.W. Greene, "Protective Groups in Organic Synthesis" beschrieben. Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure umsetzt. Stereoisomerengemische, insbesondere Diastereomerengemische, die bei Verwendung racemischer Aminosäuren A oder B anfallen, können in an sich bekannter Weise durch fraktionierte Kristallisation oder durch Chromatographie getrennt werden.

Die erfindungsgemäßen Verbindungen der Formel I weisen enzymhemmende Eigenschaften auf; insbesondere hemmen sie die Wirkung des natürlichen Enzyms Renin. Renin ist ein proteolytisches Enzym aus der Klasse der Aspartyl-Proteasen, welches als Folge verschiedener Stimuli (Volumendepletion, Natriummangel, β-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, gereinigtes Humanrenin) gemessen.

**1. Testprinzip**

Z.B. Humanplasma, welches sowohl Renin als auch Angiotensinogen enthält, wird bei 37° C mit der zu testenden Verbindung inkubiert. Dabei wird aus Angiotensinogen unter der Einwirkung von Renin Angiotensin I freigesetzt, das anschließend mit einem handelsüblichen Radioimmunoassay gemessen werden kann. Diese Angiotensin-Freisetzung wird durch Renin-Inhibitoren gehemmt.

**2. Gewinnung des Plasmas**

Das Blut wird von freiwilligen Probanden gewonnen (ca. 0,5 l pro Person; Bluko-Entnahmegerät der Fa. ASID Bonz und Sohn, Unterschleißheim) und in teilweise evakuierten Flaschen unter Eiskühlung aufgefangen. Die Gerinnung wird durch Zugabe von EDTA (Endkonzentration 10 mM) verhindert. Nach dem Zentrifugieren (Rotor HS 4 (Sorvall), 3 500 Upm, 0-4° C, 15 min; wiederholen, falls erforderlich) wird das Plasma vorsichtig abpipettiert und in geeigneten Portionen bei -30° C eingefroren. Für den Test werden nur Plasmen mit ausreichend hoher Reninaktivität verwendet. Plasmen mit niedriger Reninaktivität werden durch eine Kältebehandlung (-4° C, 3 Tage) aktiviert (Prorenin → Renin).

**3. Durchführung des Tests**

Angiotensin I wird mit dem Renin-Maia®-Kit (Serono Diagnostics S.A., Coinsins, Schweiz) bestimmt. Die Inkubation des Plasmas wird nach der dort angegebenen Anleitung durchgeführt:
Inkubationsansatz: 1000 µl Plasma (bei 0-4° C aufgetaut)
100 µl Phosphatpuffer (pH 7,4) Zusatz von $10^{-4}$ M Ramiprilat)
10 µl PMSF-Lösung
10 µl 0,1 % Genapol PFIC
12 µl DMSO bzw. Testpräparat

Die Testpräparate werden i.a. $10^{-2}$ M in 100 % Dimethylsulfoxid (DMSO) gelöst und mit DMSO entsprechend verdünnt; der Inkubationsansatz enthält max. 1 % DMSO.

Die Ansätze werden in Eis gemischt und für 1 Stunde zur Inkubation in ein Wasserbad (37° C) gestellt. Aus einem zusätzlichen Ansatz ohne Inhibitor werden ohne weitere Inkubation insgesamt 6 Proben (jeweils 100 µl) zur Bestimmung des Ausgangs-Angiotensin I-Gehaltes des verwendeten Plasmas entnommen.

Die Konzentrationen der Testpräparate werden so gewählt, daß etwa der Bereich von 10-90 % Enzymhemmung abgedeckt ist (mindestens fünf Konzentrationen). Am Ende der Inkubationszeit werden aus jedem Ansatz drei 100 µl-Proben in vorgekühlten Eppendorf-Gefäßen auf Trockeneis eingefroren und bei ca. -25° C für die Angiotensin I-Bestimmung aufbewahrt (Mittelwert aus drei Einzelproben).

**Angiotensin I-Radioimmunoassay (RIA)**

Es wird exakt die Gebrauchsanweisung des RIA-Kits (Renin-Maia®-Kit, Serono Diagnostics S.A., Coinsins, Schweiz) befolgt.

Die Eichkurve umfaßt den Bereich von 0,2 bis 25,0 ng Angiotensin I pro ml. Der Basis-Angiotensin I-Gehalt des Plasmas wird von allen Meßwerten abgezogen. Die Plasma-Renin-Aktivität (PRA) wird als ng Ang I/ml x Stunde angegeben. PRA-Werte in Gegenwart der Testsubstanzen werden auf einen Ansatz ohne Inhibitor (= 100 %) bezogen und als % Restaktivität angegeben. Aus der Auftragung von % Restaktivität gegen die Konzentration (M) des Testpräparates (logarithmische Skala) wird der $IC_{50}$-Wert abgelesen.

Die in der vorliegenden Erfindung beschriebenen Verbindungen der allgemeinen Formel I zeigen in

dem in-vitro-Test Hemmwirkungen bei Konzentrationen von etwa $10^{-5}$ bis $10^{-10}$ Mol/l.

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten (Marmosets, Rhesus-Affen) herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam, bei intraduodenaler Applikation per Gastroskop im Dosisbereich von etwa 1-50 mg/kg. Die in der vorliegenden Erfindung beschriebenen Verbindungen der allgemeinen Formel I können als Antihypertensiva, sowie zur Behandlung der Herzinsuffizienz verwendet werden.

Die HIV-Protease schneidet sich autokatalytisch aus dem GAG-POL-Polypeptid heraus und spaltet anschließend das Vorläuferpeptid p55 in die Core-Antigene p17, p24 und p14. Sie ist damit ein essentielles Enzym deren Inhibition den Lebenszyklus des Virus unterbricht und seine Vermehrung unterbindet.

In biologischen Tests zeigte sich, daß die erfindungsgemäßen Verbindungen enzyminhibitorische Wirkung haben und auch virale Enzyme wie die HIV-Protease hemmen. Besondere Bedeutung hat die HIV-Protease inhibierende Wirking, die die erfindungsgemäßen Verbindungen insbesondere zur Therapie und Prophylaxe von durch Infektion mit HIV bedingten Erkrankungen qualifiziert. Die erfindungsgemäßen Verbindungen der allgemeinen Formel I zeigen in verwendeten in-vitro-Tests Hemmwirkungen bei Konzentrationen von etwa $10^{-4}$ bis $10^{-8}$ Mol/l.

Zum Gegenstand der Erfindung gehört weiterhin die Verwendung von Verbindungen der Formel I zur Herstellung von Arzneimittel zur Bluthochdrucktherapie und der Behandlung der kongestiven Herzinsuffizienz sowie zur Therapie und Prophylaxe von Viruserkrankungen, insbesondere von Erkrankungen, die durch das HIV verursacht werden sowie die genannten Arzneimittel.

Pharmazeutische Präparate enthalten eine wirksame Menge des Wirkstoffs der Formel I zusammen mit einem anorganischem oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier-oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose-oder Mannitlösungen, oder auch eine Mischung aus den verschiedenen genannten Lösungsmitteln.

**Verzeichnis der verwendeten Abkürzungen:**

Boc tert.-Butoxycarbonyl
DC Dünnschichtchromatographie
DCC Dicyclohexylcarbodiimid
DNP 2,4-Dinitrophenyl
DMF Dimethylformamid
DMSO Dimethylsulfoxid
Fmoc 9-Fluorenylmethoxycarbonyl
EE Essigsäureethylester
FAB Fast atom bombardment
HOBt 1-Hydroxybenzotriazol
Iva Isovaleryl

M Molekularpeak
MeOH Methanol
MS Massenspektrum
R.T. Raumtemperatur
Schmp. Schmelzpunkt
Thi β-2-Thienylalanin
THF Tetrahydrofuran
Z Benzyloxycarbonyl

Die sonstigen für Aminosäuren verwendeten Abkürzungen entsprechen dem in der Peptidchemie üblichen Drei-Buchstaben-Code wie er z.B. in Eur. J. Biochem. 138, 9-37 (1984) beschrieben ist. Falls nicht ausdrücklich anders angegeben, handelt es sich immer um Aminosäuren der L-Konfiguration.

Die nachstehenden Beispiele dienen zur Erläuterung der vorliegenden Erfindung, ohne daß diese darauf beschränkt wäre.

**Beispiel 1**

N-(2-Amino-thiazol-4-yl-acetyl)-L-Phe-L-Nva-(1-S-cyclohexylmethyl-2-S-hydroxy-5-(2-pyridyl))-n-pentylamid

100 mg N-(N-Triphenylmethyl-2-amino-thiazol-4-yl-acetyl)-L-Phe-L-Nva-(1-S-cyclohexylmethyl-2-S-hydroxy-5-(2-pyridyl))-n-pentylamid wurden 5 Stunden bei R.T. in einem Gemisch aus Ameisensäure/Wasser 5:1 gerührt. Danach wurde vom ausgefallenen Triphenylmethanol abgesaugt, mit Wasser nachgewaschen und die wäßrige Lösung im Vakuum eingeengt. Der Rückstand wurde in Essigsäureethylester aufgenommen und zweimal mit verdünnter $NaHCO_3$-Lösung neutralgewaschen, danach mit gesättigter NaCl-Lösung gewaschen, über $MgSO_4$ getrocknet, vom Trockenmittel abfiltriert und eingeengt. Die Ausbeute betrug 57 mg.
Schmelzpunkt: 87 °C
MS (FAB): 664 ($M^+$ + 1)

**Beispiel 2**

N-(N-Triphenylmethyl-2-amino-thiazol-4-yl-acetyl)-L-Phe-L-Nva-(1-S-cyclohexylmethyl-2-S-hydroxy-5-(2-pyridyl))-n-pentylamid

262 mg N-(N-Triphenylmethyl-2-amino-thiazol-4-yl-acetyl)-L-Phenylalanin wurden zusammen mit 81 mg HOBt, 109 mg DCC und 67 µl NEM in 5 ml DMF gelöst und für 1 Stunde bei R.T. gerührt. Danach wurden 180 mg L-Norvalin-(1-S-Cyclohexylmethyl-2-S-hydroxy-5-(2-pyridyl))-n-pentylamid gelöst in 2 ml DMF dazugegeben. Die Reaktion wurde 48 Stunden bei R.T. gerührt. Danach wurde mit Wasser verdünnt, vom ausgefallenen Dicyclohexylharnstoff abfiltriert und die Lösung im Vakuum eingeengt. Der Rückstand wurde mit Essigsäureethylester aufgenommen und dreimal mit gesättigter $NaHCO_3$-Lösung, zweimal mit gesättigter NaCl gewaschen, anschließend über $MgSO_4$ getrocknet und eingeengt. Nach chromatographischer Reinigung auf Kieselgel ($CH_2Cl_2$/MeOH 20:1) wurden 210 mg Produkt erhalten.
Schmelzpunkt: 75 °C
MS (FAB): 906 ($M^+$ + 1)

**Beispiel 3**

N-(N-Triphenylmethyl-2-amino-thiazol-4-yl-acetyl)-L-phenylalanin

a) 3,2 g N-Triphenylmethyl-2-amino-thiazol-4-yl-essigsäure, 1,34 g HOBt, 1,81 g DCC, 1,43 g L-Phe-OMe und 1 ml N-Ethylmorpholin wurden nacheinander in 30 ml DMF (absolut) gelöst und über Nacht gerührt. Nach 24 Stunden wurde mit wenig Wasser versetzt, vom ausgefallenen Dicyclohexylharnstoff abfiltriert und die erhaltene Lösung im Vakuum eingeengt. Der Rückstand wurde in Essigsäureethylester

aufgenommen und dreimal mit verdünnter $NaHCO_3$-Lösung, zweimal mit 10 %iger Citronsäure, zweimal mit gesättigter NaCl-Lösung gewaschen, die organische Phase über $MgSO_4$ getrocknet und danach eingeengt. Säulenchromatographie über Kieselgel mit Cyclohexan/EE ergab 3,6 g des Methylesters als Öl.

b) 3,5 g des unter a) erhaltenen Methylesters wurden in 30 ml Dioxan-Wasser-Gemisch (1:1) mit äquimolarer Menge 1N-Natronlauge 3 Stunden bei R.T. gerührt. Danach wurde die Reaktionslösung im Vakuum vom Dioxan befreit, mit Diethylether extrahiert, die wäßrige Phase auf pH 3 angesäuert und mit Essigsäureethylester intensiv dreimal extrahiert. Die organische Phase wurde über $MgSO_4$ getrocknet, vom Trockenmittel abfiltriert und im Vakuum eingeengt. Die Ausbeute des farblosen Feststoffs betrug 2,7 g.
Schmelzpunkt: 205-208 °C

**Beispiel 4**

N-Tert.-butoxycarbonyl-norvalyl-(1-S-cyclohexylmethyl-2-S-hydroxy-5-(2-pyridyl))-n-pentylamid

2,1 g N-Tert.butoxycarbonyl-norvalin, 1,6 g HOBt, 2,1 g DCC und 1,4 ml N-Ethylmorpholin wurden in 50 ml DMF (absolut) gelöst. Zu dieser Mischung wurde bei 0 °C 2,6 g 2-S-Amino-1-cyclohexyl-3-S-hydroxy-6-(2-pyridyl)-hexan gelöst in 5 ml DMF zugegeben. Die Lösung wurde 48 Stunden bei R.T. gerührt. Danach wurde mit 5 ml Wasser versetzt, vom ausgefallenen Dicyclohexylharnstoff abfiltriert und mit 150 ml Essigsäureethylester verdünnt. Diese Phase wurde dreimal mit gesättigter $NaHCO_3$-Lösung, zweimal mit gesättigter NaCl-Lösung und zweimal mit Wasser extrahiert. Die verbleibende organische Phase wurde über $MgSO_4$ getrocknet und im Vakuum eingeengt und über Kieselgel mit $CH_2Cl_2$/MeOH chromatographiert. Es wurden 3,56 g als zähes Öl erhalten.
Drehwert: $\alpha_D^{22}$ = -47,4 °C (c = 1,135, Methanol)
MS (FAB): 476 ($M^+$ + 1)

**Beispiel 5**

L-Nva-(1-S-Cyclohexylmethyl-2-5-hydroxy-5-(2-pyridyl)-n-pentylamid

200 mg der in Beispiel 4 beschriebenen Verbindung wurden in 3 ml Trifluoressigsäure bei 0 °C aufgelöst und 30 Minuten gelöst. Nach Aufwärmen auf R.T. wurde die überschüssige Trifluoressigsäure im Vakuum abgezogen, der Rückstand in Essigsäureethylester gelöst und dreimal mit verdünnter $NaHCO_3$-Lösung extrahiert. Die organische Phase wurde mit gesättigter NaCl-Lösung und Wasser gewaschen, über $MgSO_4$ getrocknet, vom Trockenmittel abfiltriert und eingeengt. Man erhielt ein Öl. Das auf diese Weise N-terminal entschützte Aminosäurederivat wurde sehr rasch für weitere Reaktionen verwendet.
MS (FAB): 376 ($M^+$ + 1)

**Beispiel 6**

N-(S-(4-Pyridyl)-mercaptoacetyl)-L-phenylalanin

Die Titelverbindung wurde hergestellt aus (4-Pyridyl)-mercaptoessigsäure und L-Phenylalanin-methylester nach dem in Beispiel 3 beschriebenen Verfahren.
Schmelzpunkt: 199 - 201 °C
$^1$H-NMR (60 MHz, $d_6$-DMSO): $\delta$ = 3,05 (2H, $CH_2$; 3,8 (2H, $CH_2CO$); 4,5 (m, 1H, C-$H_\alpha$), 7,30 (s, 5H, Phenyl); 8,0-8,7 (m, 4H, Pyridyl) ppm
MS(DCI): 317 ($M^+$ + 1).

**Beispiel 7**

N-(S-(4-Pyridyl)-mercaptoacetyl)-L-Phe-L-Nva-(1-S-cyclohexylmethyl-2-S-hydroxy-5-(2-pyridyl))-n-pentylamid

Die Titelverbindung wurde hergestellt aus dem in Beispiel 6 beschriebenen Phenylalanin-Derivat und aus dem in Beispiel 5 beschriebenen Norvalin-Derivat nach dem in Beispiel 2 beschriebenen Verfahren.
MS (FAB): 674 ($M^+ + 1$)

**Beispiel 8**

N-(2-Pyridyl)-ethoxycarbonyl-L-Phe-L-Nva-OH

Die Titelverbindung wurde hergestellt aus N-(2-Pyridyl)-ethoxycarbonyl-L-phenylalanin und L-Nva-OMe nach dem im Beispiel 3a) beschriebenen Verfahren und anschließender in Beispiel 3b) beschriebener Esterverseifung.
MS (FAB): 414 ($M^+ + 1$)

**Beispiel 9**

N-(2-Pyridyl)-ethoxycarbonyl-L-Phe-L-Nva-(1-S-cyclohexylmethyl-2-S-hydroxy-5-(2-pyridyl))-n-pentylamid

Die Titelverbindung wurde erhalten aus dem in Beispiel 8 beschriebenen Dipeptid und 2-S-Amino-1-S-cyclohexyl-3-S-hydroxy-6-(2-pyridyl)-hexan nach dem in Beispiel 4 beschriebenen Verfahren.
MS (FAB): 672 ($M^+ + 1$)

**Beispiel 10**

N-(3-Pyridyl)-ethoxycarbonyl-L-Phe-L-Nva-(1-S-cyclohexylmethyl-2-S-hydroxy-5-(2-pyridyl))-n-pentylamid

Die Titelverbindung wurde hergestellt aus N-(3-Pyridyl)-ethoxycarbonyl-L-Phe und der in Beispiel 5 beschriebenen Verbindung nach dem in Beispiel 2 beschriebenen Verfahren. Schmelzpunkt: 46-50 °C
MS (FAB): 672 ($M^+ + 1$)

**Beispiel 11**

N-(4-Pyridyl)-ethoxycarbonyl-L-Phe-L-Nva-(1-S-cyclohexylmethyl-2-S-hydroxy-5-(2-pyridyl))-n-pentylamid

Die Titelverbindung wurde erhalten aus N-(4-Pyridyl)-ethoxycarbonyl-L-Phe und der in Beispiel 5 beschriebenen Verbindung nach dem in Beispiel 2 beschriebenen Verfahren.
MS (FAB): 672 ($M^+ + 1$)

**Beispiel 12**

Fmoc-His(Trt)-(1-S-cyclohexylmethyl-2S-hydroxy-5-(2-pyridyl))-n-pentylamid

1,2 g Fmoc-His(Trt)-OH, 340 mg HOBt, 445 mg DCC und 0,3 ml NEM wurden in 11 ml DMF gelöst und 1 Stunde bei R.T. gerührt. Diese Mischung wurde mit 580 mg 2-S-Amino-1-S-cyclohexyl-3-S-hydroxy-6-(2-pyridyl)hexan gelöst in 4 ml DMF versetzt und über Nacht gerührt. Nach Zugabe von 5 ml Wasser wurde vom ausgefallenen Harnstoff abfiltriert und mit 100 ml Essigsäureethylester aufgenommen. Die organische Phase wurde dreimal mit gesättigter $NaHCO_3$-Lösung, zweimal mit gesättigter NaCl-Lösung gewaschen,

anschließen über $MgSO_4$ getrocknet und nach Filtration im Vakuum eingeengt. Der Rückstand wurde über Kieselgel chromatographiert ($CH_2Cl_2$/MeOH). Die Ausbeute der genannten Verbindung betrug 1,06 g.
Schmelzpunkt: 85 °C

**Beispiel 13**

H-His(Trt)-(1-S-cyclohexylmethyl-2-S-hydroxy-5-(2-pyridyl))-n-pentylamid

500 mg der in Beispiel 12 beschriebenen Verbindung wurden in 5 ml DMF (absolut) zusammen mit 0,6 ml Diethylamin gelöst und 20 Minuten bei R.T. gerührt. Danach wurde das Lösungsmittel im Hochvakuum abgezogen und der verbleibende Rückstand über Kieselgel mit $CH_2Cl_2$/MeOH chromatographiert. Auf diese Weise wurden 320 mg der Titelverbindung isoliert.
MS (FAB): 656 ($M^+$ +1)

**Beispiel 14**

N-(2-Pyridyl)-ethoxycarbonyl-L-Phe-L-His(Trt)-(1-S-cyclohexylmethyl-2-S-hydroxy-5-(2-pyridyl))-n-pentylamid

157 mg 2-Pyoc-L-Phe, 85 mg HOBt, 113 mg DCC und 70 µl NEM wurden in 5 ml DMF gelöst und mit einer Lösung von 310 mg der Verbindung aus Beispiel 13 in 3 ml DMF versetzt. Man läßt 58 Stunden rühren und arbeitet wie in Beispiel 12 beschrieben auf. Es wurden 265 mg der Titelverbindung isoliert.
MS (FAB): 953 ($M^+$ +1)

**Beispiel 15**

N-(2-Pyridyl)-ethoxycarbonyl-L-Phe-L-His-(1-S-cyclohexylmethyl-2-S-hydroxy-5-(2-pyridyl))-n-pentylamid

115 mg der Verbindung aus Beispiel 14 wurden in 2,5 ml Trifluoressigsäure 1,5 Stunden gerührt. Danach wurde die überschüssige Säure im Vakuum entfernt und der Rückstand in Essigsäureethylester gelöst und mit verdünnter $NaHCO_3$-Lösung neutralisiert. Die Essigester-Lösung wurde über $MgSO_4$ getrocknet, dann abfiltriert und im Vakuum eingeengt. Chromatographie des Rückstandes über Kieselgel mit $CH_2Cl_2$/MeOH ergab 57 mg gewünschtes Produkt.
Schmelzpunkt: 68 °C
MS (FAB): 710 ($M^+$ +1)

**Beispiel 16**

2-Pyoc-L-Methionin

6 g 2-(2-Pyridyl)-ethyl-p-nitrophenylcarbonat und 2,5 g L-Met-OMe wurden in 60 ml Acetonitril gelöst, bis zum Erreichen des pH-Wertes 8,5-9,0 mit 1N-NaOH-Lauge versetzt und bis zur vollständigen Reaktion gerührt (DC-Kontrolle). Anschließend wurde das Acetonitril im Vakuum abgezogen und die wäßrige Lösung des Reaktionsgemisches bei pH 9, bei pH 6 und nach weiterem Ansäuern mit 1N-HCl bei pH 3 mit Diethylether extrahiert. Die letzte Extraktion ergab das gewünschte Produkt, das durch Säulenchromatographie auf Kieselgel mit $CH_2Cl_2$/EE gereinigt in einer Ausbeute von 2,43 g erhalten wurde. 2,2 g des so erhaltenen Öls wurden in 30 ml Ethanol/Wasser 2:1 gelöst und mit 585 mg festem NaOH versetzt. Nach 2 Stunden wurde der Ethanol im Vakuum abdestilliert, die wäßrige Lösung auf pH 3 eingestellt und ebenfalls im Vakuum eingeengt. Der verbleibende Rückstand wurde mit Aceton verrührt und die organische Lösung von den ungelösten Bestandteilen abgetrennt. Nach Trocknen und Einengen der Lösung wurden 1,89 g der

Titelverbindung erhalten.
Drehwert: $\alpha_D^{20}$ = 9,4° (c = 1, Ethanol)

**Beispiel 17**

N-(2-Pyridyl)-ethoxycarbonyl-L-Met-L-Nva-(1-S-cyclohexylmethyl-2-S-hydroxy-5-(2-pyridyl))-n-pentylamid

167 mg 2-Pyoc-Met-OH und 190 mg der Verbindung aus Beispiel 5 wurden nach dem Verfahren des Beispiels 2 umgesetzt. Nach beschriebener Aufarbeitung und Chromatographie wurden 64 mg der Titelverbindung isoliert.
Schmelzpunkt: 104-105 °C
MS (FAB): 657 ($M^+$ + 1)

**Beispiel 18**

4-Pyridylmethyl-4-nitrophenylcarbonat

20,3 g Chlorameisensäure-p-nitrophenylester wurden in 150 ml $CH_2Cl_2$ (absolut) gelöst und unter $N_2$ auf 0 °C gekühlt. Dazu gab man 520 mg 4-Dimethylaminopyridin (DMAP) und tropfte anschließend 10 g 4-Hydroxymethylpyridin in 50 ml $CH_2Cl_2$ (absolut) zu dieser Lösung. Man ließ bei R.T. über Nacht rühren und filtrierte die ausgefallenen Kristalle ab. Die Kristalle wurden mit frischem $CH_2Cl_2$ in der Hitze umkristallisiert und nach dem Abkühlen erneut abfiltriert.
Ausbeute: 17,5 g.
Schmelzpunkt: 150-154 °C

**Beispiel 19**

N-4-Pyridylmethoxycarbonyl-L-Phenylalanin

8 g 4-Pyridylmethyl-4-nitrophenylcarbonat und 4,82 g L-Phenylalanin wurden in 350 ml Acetonitril/Wasser 1:1 gelöst und mit etwa 45 ml 2N-NaOH bis pH 10 versetzt. Die Mischung rührte über Nacht und wurde von Acetonitril im Vakuum befreit. Die wäßrige Lösung wurde auf pH 6 gebracht und dreimal mit Diethylether gewaschen, danach auf pH 1 eingestellt und bis zum festen Rückstand eingeengt. Dieser wurde in Wasser gelöst und mit Essigester intensiv extrahiert, danach auf pH 2 eingestellt und der ausgefallene Niederschlag abgesaugt. Der Niederschlag betrug nach dem Trocknen 7,3 g.
Schmelzpunkt: 195-197 °C

Analog der in Beispiel 19 beschriebenen Verbindung wurden folgende Verbindungen hergestellt:

**Beispiel 20**

N-(2-(N-Phtalimidyl)-ethoxycarbonyl)-L-Phenylalanin

Schmelzpunkt: 65-72 °C

**Beispiel 21**

N-(3-Pyridylethoxycarbonyl)-L-Phenylalanin • Hydrochlorid

Schmelzpunkt: 105 °C

**Beispiel 22**

N-(2-Pyridylethoxycarbonyl)-L-Methioninsulfon • Hydrochlorid

Schmelzpunkt: 48 °C

**Beispiel 23**

N-(4-Pyridylethoxycarbonyl)-L-phenylalanin

Schmelzpunkt: 196-204 °C (Zersetzung)

**Beispiel 24**

N-(N-Tert.-butoxycarbonyl-4-piperidyl)-ethoxycarbonyl-L-phenylalanin

MS (DCI): 421 ($M^+$ +1)

**Beispiel 25**

N-(N-Tert.-butoxycarbonyl-2-piperidyl)-ethoxycarbonyl-L-phenylalanin

Schmelzpunkt: 52 °C

**Beispiel 26**

N-(2-(4-Morpholino)-ethoxycarbonyl)-L-phenylalanin

MS (DCI): 323 ($M^+$ +1) Schmelzpunkt: 66 °C (Sublimation)

**Beispiel 27**

N-(2-(N-Methylpyrrolidin-2-yl)-ethoxycarbonyl)-L-phenylalanin

MS (DCI): 321 ($M^+$ +1)

**Beispiel 28**

N-(2-(4-Tert.-butoxycarbonylpiperazin-1-yl)-ethoxycarbonyl)-L-phenylalanin

MS (DCI): 421 ($M^+$ +1) Schmelzpunkt: 85 °C

Analog der in Beispiel 17 beschriebenen Verbindung wurden folgende Verbindungen hergestellt:

**Beispiel 29**

N-(2-(N-Phtalimidyl)-ethoxycarbonyl)-L-Phe-L-Nva-(1-S-cyclohexylmethyl-2-S-hydroxy-5-(2-pyridyl))-n-pentylamid

Die Titelverbindung wurde hergestellt aus den Verbindungen der Beispiele 20 und 5 nach dem in

Beispiel 2 beschriebenen Verfahren.
Schmelzpunkt: 57-62 °C
MS (FAB): 740 ($M^+$ + 1)

**Beispiel 30**

N-(3-Pyridylethoxycarbonyl)-L-Phe-L-His-(1-S-cyclohexylmethyl-2-S-hydroxy-5-(2-pyridyl))-n-pentylamid

MS (FAB): 709 ($M^+$ + 1)

**Beispiel 31**

N-(2-Morpholin-4-yl)-ethoxycarbonyl-L-Phe-L-Nva-(1-S-cyclohexylmethyl-2-S-hydroxy-5-(2-pyridyl))-n-pentylamid

MS (FAB): 680 ($M^+$ + 1)

**Beispiel 32**

N-(2-(N-Methylpyrrolidin-2-yl)-ethoxycarbonyl)-L-Phe-L-Nva-(1-S-cyclohexylmethyl-2-S-hydroxy-5-(2-pyridyl))-n-pentylamid

Schmelzpunkt: 115-125 °C
MS (FAB): 678 ($M^+$ + 1)
$R_F$ 0,52 ($CH_2Cl_2/CH_3OH/H_2O/CH_3COOH$ 100/50/10/5)

**Beispiel 33**

N-(2-(4-Tert.-butoxycarbonylpiperazin-1-yl)-ethoxycarbonyl)-L-Phe-L-Nva-(1-S-cyclohexylmethyl-2-S-hydroxy-5-(2-pyridyl))-n-pentylamid

MS (FAB): 779 ($M^+$ + 1)

**Beispiel 34**

N-(2-(2-Pyridyl)-ethoxycarbonyl)-L-Methioninsulfon-L-norvalin-(1-S-cyclohexylmethyl-2-S-hydroxy-5-(2-pyridyl))-n-pentylamid

Schmelzpunkt: 79-81 °C
MS (FAB): 689 ($M^+$ + 1)

**Beispiel 35**

N-(2-(N-Tert.butoxycarbonyl-2-piperidyl)ethoxycarbonyl)-L-Phe-L-Nva-(1-S-Cyclohexylmethyl-2-S-hydroxy-5-(2-pyridyl)-pentylamid

Die Titelverbindung wurde hergestellt aus den Verbindungen der Beispiele 5 und 24 nach dem in Beispiel 2 beschriebenen Verfahren.
MS (FAB): 778 ($M^+$ + 1)
Schmelzpunkt: 59-61 °C

**Beispiel 36**

N-(2-(2-Piperidyl)-ethoxycarbonyl)-L-Phe-L-Nva-(1-S-cyclohexylmethyl-2-S-hydroxy-5-(2-pyridyl)-pentylamid

Die Titelverbindung wurde hergestellt aus der in Beispiel 34 beschriebenen Verbindung nach dem in Beispiel 5 beschriebenen Verfahren mit anschließender chromatographischer Reinigung auf Kieselgel.
MS (FAB): 678 ($M^+$ + 1)
$R_F$ 0,30 ($CH_2Cl_2$/MeOH 9:1)

**Beispiel 37**

N-(2-(N-Tert.butoxycarbonyl-4-piperidyl)-ethoxycarbonyl)-L-Phe-L-Nva-(1-S-cyclohexylmethyl-2-S-hydroxy-5-(2-pyridyl)-pentylamid

Die Titelverbindung wurde hergestellt aus den Verbindungen der Beispiele 23 und 5 nach dem in Beispiel 2 beschriebenen Verfahren.
MS (FAB): 778 ($M^+$ + 1)
Schmelzpunkt: 65-72 °C

**Beispiel 38**

N-(2-(4-Piperidyl)-ethoxycarbonyl)-L-Phe-L-Nva-(1-S-cyclohexylmethyl-2-S-hydroxy-5-(2-pyridyl)-pentylamid

Die Titelverbindung wurde hergestellt aus der in Beispiel 36 beschriebenen Verbindung nach dem in Beispiel 5 beschriebenen Verfahren mit anschließender chromatographischer Reinigung auf Kieselgel.
MS (FAB): 678 ($M^+$ + 1)
$R_F$ 0,22 ($CH_2CH_2$/Methanol 9:1)

**Beispiel 39**

N-(2-(N-Tert.butoxycarbonyl-2-piperidyl)-ethoxycarbonyl)-L-Phe-L-His-(1-S-cyclohexylmethyl-2-S-hydroxy-5-(2-pyridyl))-pentylamid

Schmelzpunkt: 98 °C Zersetzung
MS (FAB): 817 ($M^+$ + 1)
$R_F$ 0,33 ($CH_2Cl_2$/$CH_3OH$ 9:1)

**Beispiel 40**

N-(2-(2-Piperidyl)-ethoxycarbonyl)-L-Phe-L-His-(1-S-cyclohexylmethyl-2-S-hydroxy-5-(2-pyridyl))-pentylamid

MS (FAB): 717 ($M^+$ +)

**Beispiel 41**

N-(2-Morpholin-4-yl)-ethoxycarbonyl-L-Phe-L-His-(1-S-cyclohexylmethyl-2-S-hydroxy-5-(2-pyridyl))-pentylamid

Schmelzpunkt: 70-81 °C

MS (FAB): 718 ($M^+$ +1)
$R_F$ 0,72 ($CH_2Cl_2/CH_3OH$ 9:1)

**Beispiel 42**

N-(2,2,5,5-Tetramethyl-1,3-thiazolidin-4-yl-carbonyl)-L-phenylalanyl-L-norvalyl-(1-S-cyclohexylmethyl-2-S-hydroxy-5-(2-pyridyl))-pentylamid

Die Titelverbindung wurde hergestellt aus der in Beispiel 5 beschriebenen Verbindung und N-(2,2,5,5-Tetramethyl-1,3-thiazolidin-4-yl-carbonyl)-L-phenylalanin nach dem in Beispiel 2 becshriebenen Verfahren.
MS (FAB) 695 ($M^+$1)
$R_F$ 0,51 ($CH_2Cl_2/CH_3OH$)

**Beispiel 43**

N-(2-(4-Tert.-butoxycarbonylpiperazin-1-yl)-ethoxycarbonyl)-L-Phe-L-His-(1-S-cyclohexylmethyl-2-S-hydroxy-5-(2-pyridyl))-n-pentylamid

Hergestellt aus Beispiel 13 und 28 nach Verfahren Beispiel 2.
MS (FAB) 817 ($M^+$ +1)
Schmelzpunkt: 82-89 °C

**Beispiel 44**

N-(2-Piperazin-1-yl)-ethoxycarbonyl)-L-Phe-L-His-(1-S-cyclohexylmethyl-2-S-hydroxy-5-(2-pyridyl))-n-pentylamid

Hergestellt aus Beispiel 43 nach Verfahren Beispiel 5.
MS (FAB) 717 ($M^+$ +1)

**Beispiel 45**

N-Nicotinoyl-L-Phe-L-His-(1-S-cyclohexylmethyl-2-S-hydroxy-5-(2-pyridyl))-pentylamid

Die Titelverbindung wurde aus N-Nicotinoyl-L-Phenylalanin und der in Beispiel 5 beschriebenen Verbindung nach dem Verfahren aus Beispiel 14 hergestellt. Das auf diese Weise erhaltene Produkt wurde mit $CF_3COOH$ nach dem Verfahren des Beispiels 15 behandelt. Nach Chromatographie betrug die Ausbeute 412 mg.
MS (FAB) 666 ($M^+$ +1)
Schmelzpunkt: 103-110 °C
$R_F$ 0,18 ($CH_2Cl_2/CH_3OH$ 9:1)

**Ansprüche**

1. Verbindung der Formel I

$$R^1-\overset{\overset{\large O}{\|}}{C}-A-B-NH-\overset{\overset{\large R^2}{|}}{CH}-\overset{\overset{\large OH}{|}}{CH}-\overset{\overset{\large R^3}{|}}{CH}-R^4 \qquad (I),$$

in welcher
$R^1$ Het-($C_1$-$C_6$)-alkyl,
Het-($C_1$-$C_6$)-alkoxy,
Het-($C_3$-$C_8$)-cycloalkyl,
Het-($C_3$-$C_8$)-cycloalkyl-($C_1$-$C_4$)-alkyl,
Het-($C_3$-$C_8$)-cycloalkoxy,
Het-($C_3$-$C_8$)-cycloalkoxy-($C_1$-$C_4$)-alkyl,
Het-mercapto-($C_1$-$C_6$)-alkyl,
Het-mercapto-($C_3$-$C_8$)-cycloalkyl,
Het-mercapto-($C_3$-$C_8$)-cycloalkyl-($C_1$-$C_4$)-alkyl,
Het-mercapto-($C_1$-$C_6$)-alkoxy,
Het-mercapto-($C_3$-$C_8$)-cycloalkoxy bedeutet,
wobei Het für einen 5- bis 7-gliedrigen monocyclischen oder 8- bis 10-gliedrigen bicyclischen heterocyclischen Ring steht, der benzannelliert, aromatisch, teilhydriert oder vollständig hydriert sein kann, der als Heteroelemente einen, zwei oder drei, gleiche oder verschiedene Reste aus der Gruppe N, O, S, NO, SO, $SO_2$ enthalten kann und der durch einen oder zwei, gleiche oder verschiedene Reste aus der Gruppe ($C_1$-$C_6$)-Alkyl, ($C_1$-$C_6$)-Alkoxy, ($C_1$-$C_6$)-Alkoxycarbonyl, Hydroxy, Halogen, Amino, Mono- oder Di-($C_1$-$C_6$)-alkylamino, Oxido substituiert sein kann;
$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl oder ($C_6$-$C_{14}$)-Aryl-($C_1$-$C_4$)-alkyl bedeutet;
$R^3$ Wasserstoff, ($C_1$-$C_{10}$)-Alkyl, ($C_6$-$C_{14}$)-Aryl, ($C_6$-$C_{14}$)-Aryl-($C_1$-$C_4$)-alkyl oder Hydroxy bedeutet;
$R^4$ einen Rest der Formel II bedeutet,
$(CH_2)_m$-$CHR^5$-D (II)
wobei
$R^5$ für Wasserstoff, ($C_1$-$C_7$)-Alkyl, ($C_1$-$C_5$)-Alkoxy, ($C_1$-$C_5$)-Alkylthio, ($C_1$-$C_5$)-Alkylamino, Hydroxy, Azido, Fluor, Chlor, Brom, Jod steht,
D einen Rest Het bedeutet, wobei
Het einen 5- bis 7-gliedrigen heterocyclischen Ring steht, der benzannelliert, aromatisch, teilhydriert oder vollständig hydriert sein kann, der als Heteroatome einen oder zwei gleiche oder verschiedene Reste aus der Gruppe N, O, S, NO, SO, $SO_2$ enthalten kann und der durch einen oder zwei gleiche oder verschiedene Reste aus der Gruppe ($C_1$-$C_4$)-Alkyl, ($C_1$-$C_4$)-Alkoxy, Hydroxy, Halogen, Amino, Mono- oder Di-($C_1$-$C_4$)-alkylamino, $CF_3$ substituiert sein kann, und m 0, 1, 2, 3 oder 4 bedeutet;
A und B unabhängig voneinander einen N-terminal mit $R^1$ bzw. A und C-terminal mit B bzw. NH-$CHR^2$-CHOH-$CHR^3$-$R^4$ verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, 2-Amino-4-(3-thienyl)-buttersäure, 3-(2-Thienyl)-serin, (Z)-Dehydrophenylalanin, (E)-Dehydrophenylalanin, 1,3-Dioxolan-2-yl-alanin, N-Pyrrolylalanin und 1-, 3- oder 4-Pyrazolylalanin bedeuten,
sowie deren physiologisch verträgliche Salze.

2. Verbindung der Formel I, gemäß Anspruch 1, in welcher
$R^1$ wie in Anspruch 1 definiert ist;
$R^2$ Isobutyl, Benzyl oder Cyclohexylmethyl bedeutet;
$R^3$ Wasserstoff, ($C_1$-$C_5$)-Alkyl, ($C_6$-$C_{10}$)-Aryl, ($C_6$-$C_{10}$)-Aryl-($C_1$-$C_4$)-alkyl oder Hydroxy bedeutet;
$R^4$ einen Rest der Formel II bedeutet, worin
$R^5$ für Wasserstoff, ($C_1$-$C_4$)-Alkyl, ($C_1$-$C_4$)-Alkoxy, ($C_1$-$C_4$)-Alkylthio, ($C_1$-$C_4$)-Alkylamino, Hydroxy, Azido, Fluor, Chlor, Brom oder Jod steht,
D wie in Anspruch 1 definiert ist, und
m 0, 1 oder 2 bedeutet; und
A und B unabhängig voneinander einen bivalenten Rest aus der Reihe
Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, Norleucin, Cystein, 5-Methyl-Cystein, 1,2,3,4-Tetrahydroisochinolin-3-carbonsäure, Homophenylalanin, 2-Amino-4-(2-thienyl)-buttersäure, 2-Amino-4-(3-thienyl)-buttersäure, 3-(2-Thienyl)-serin, (Z)-Dehydrophenylalanin, (E)-Dehydrophenylalanin, 1,3-Dioxolan-2-yl-alanin, N-Pyrrolylalanin und 1-, 3- oder 4-Pyrazolylalanin, bedeuten,
sowie deren physiologisch verträgliche Salze.

3. Verbindung der Formel I, gemäß einem oder mehreren der Ansprüche 1 bis 2, in welcher
$R^1$ Het-($C_1$-$C_4$)-alkyl,
Het-($C_1$-$C_4$)-alkoxy,
Het-($C_5$-$C_6$)-cycloalkyl,
Het-($C_5$-$C_6$)-cycloalkoxy,
Het-mercapto-($C_1$-$C_3$)-alkyl,
Het-mercapto-($C_5$-$C_6$)-cycloalkyl,
Het-mercapto-($C_1$-$C_3$)-alkoxy,
Het-mercapto-($C_5$-$C_6$)-cycloalkoxy bedeutet,
wobei Het für einen 5- bis 6-gliedrigen heterocyclischen Ring steht, wie z.B. Furanyl, Thienyl, Pyrrolyl, Imidazolyl, Isoxazolyl, Thiazolyl, Pyrazolyl, Triazolyl, Pyridyl, Pyrimidyl, Pyrazinyl, Pyridazinyl, Triazinyl, Pyrrolidyl, Piperidyl, Piperazinyl, Morpholino, Thiomorpholino, Tetrahydrofuranyl, Tetrahydropyranyl, Tetrahydrothienyl, und durch einen oder zwei gleiche oder verschiedene Reste aus der Gruppe ($C_1$-$C_4$)-Alkyl, ($C_1$-$C_4$)-Alkoxy, ($C_1$-$C_4$)-Alkoxycarbonyl, Hydroxy, Amino, Mono- oder Di-($C_1$-$C_4$)-Alkylamino und Oxido substituiert sein kann;
$R^2$ Isobutyl, Benzyl oder Cyclohexylmethyl bedeutet;
$R^3$ Wasserstoff oder Hydroxy bedeutet;
$R^4$ einen Rest der Formel II bedeutet, in welcher
$R^5$ für Wasserstoff oder Fluor steht,
D für einen 2-, 3- oder 4-Pyridinrest, einen 2-, 4- oder 5-Imidazolrest oder einen 2-Oxazolinrest steht, wobei die genannten Heterocyclen jeweils durch einen oder zwei Reste aus der Gruppe Methyl, Methoxy, Fluor, Chlor, Brom und $CF_3$ substituiert sein können, und
m 0, 1 oder 2 bedeutet; und
A und B unabhängig voneinander einen bivalenten Rest aus der Reihe
Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, Norleucin, 1,2,3,4-Tetrahydroisochinolin-3-carbonsäure, Homophenylalanin, 2-Amino-4-(2-thienyl)-buttersäure und 1-, 3- und 4-Pyrazolylalanin bedeuten,
sowie deren physiologisch verträgliche Salze.

4. Verfahren zur Herstellung einer Verbindung der Formel I, gemäß einem oder mehreren der Ansprüche 1 bis 3, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

5. Verbindung der Formel I gemäß einem oder mehreren der Ansprüche 1 bis 3 zur Anwendung als Heilmittel.

6. Verbindung der Formel I gemäß einem oder mehreren der Ansprüche 1 bis 3 zur Anwendung als Heilmittel bei der Behandlung des Bluthochdrucks.

7. Verbindung der Formel I gemäß einem oder mehreren der Ansprüche 1 bis 3 zur Anwendung als Heilmittel bei der Behandlung viraler Erkrankungen.

8. Pharmazeutische Zubereitung enthaltend eine Verbindung der Formel I gemäß einem oder mehreren der Ansprüche 1 bis 3.

Patentansprüche für folgende Vertragsstaaten: ES, GR

1. Verfahren zur Herstellung einer Verbindung der Formel I

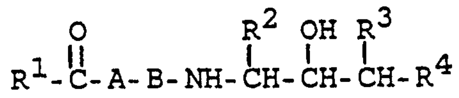

(I),

in welcher

$R^1$ Het-($C_1$-$C_6$)-alkyl,
Het-($C_1$-$C_6$)-alkoxy,
Het-($C_3$-$C_8$)-cycloalkyl,
Het-($C_3$-$C_8$)-cycloalkyl-($C_1$-$C_4$)-alkyl,
Het-($C_3$-$C_8$)-cycloalkoxy,
Het-($C_3$-$C_8$)-cycloalkoxy-($C_1$-$C_4$)-alkyl,
Het-mercapto-($C_1$-$C_6$)-alkyl,
Het-mercapto-($C_3$-$C_8$)-cycloalkyl,
Het-mercapto-($C_3$-$C_8$)-cycloalkyl-($C_1$-$C_4$)-alkyl,
Het-mercapto-($C_1$-$C_6$)-alkoxy,
Het-mercapto-($C_3$-$C_8$)-cycloalkoxy bedeutet,

wobei Het für einen 5- bis 7-gliedrigen monocyclischen oder 8- bis 10-gliedrigen bicyclischen heterocyclischen Ring steht, der benzannelliert, aromatisch, teilhydriert oder vollständig hydriert sein kann, der als Heteroelemente einen, zwei oder drei, gleiche oder verschiedene Reste aus der Gruppe N, O, S, NO, SO, $SO_2$ enthalten kann und der durch einen oder zwei, gleiche oder verschiedene Reste aus der Gruppe ($C_1$-$C_6$)-Alkyl, ($C_1$-$C_6$)-Alkoxy, ($C_1$-$C_6$)-Alkoxycarbonyl, Hydroxy, Halogen, Amino, Mono- oder Di-($C_1$-$C_6$)-alkylamino, Oxido substituiert sein kann;

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl oder ($C_6$-$C_{14}$)-Aryl-($C_1$-$C_4$)-alkyl bedeutet;

$R^3$ Wasserstoff, ($C_1$-$C_{10}$)-Alkyl, ($C_6$-$C_{14}$)-Aryl, ($C_6$-$C_{14}$)-Aryl-($C_1$-$C_4$)-alkyl oder Hydroxy bedeutet;

$R^4$ einen Rest der Formel II bedeutet,

$(CH_2)_m$-$CHR^5$-D (II)

wobei

$R^5$ für Wasserstoff, ($C_1$-$C_7$)-Alkyl, ($C_1$-$C_5$)-Alkoxy, ($C_1$-$C_5$)-Alkylthio, ($C_1$-$C_5$)-Alkylamino, Hydroxy, Azido, Fluor, Chlor, Brom, Jod steht,

D einen Rest Het bedeutet, wobei

Het einen 5- bis 7-gliedrigen heterocyclischen Ring steht, der benzannelliert, aromatisch, teilhydriert oder vollständig hydriert sein kann, der als Heteroatome einen oder zwei gleiche oder verschiedene Reste aus der Gruppe N, O, S, NO, SO, $SO_2$ enthalten kann und der durch einen oder zwei gleiche oder verschiedene Reste aus der Gruppe ($C_1$-$C_4$)-Alkyl, ($C_1$-$C_4$)-Alkoxy, Hydroxy, Halogen, Amino, Mono- oder Di-($C_1$-$C_4$)-alkylamino, $CF_3$ substituiert sein kann, und

m 0, 1, 2, 3 oder 4 bedeutet;

A und B unabhängig voneinander einen N-terminal mit $R^1$ bzw. A und C-terminal mit B bzw. NH-$CHR^2$-CHOH-$CHR^3$-$R^4$ verknüpften Rest einer Aminosäure aus der Reihe Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, β-2-Benzo[b]thienylalanin, β-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, 2-Amino-4-(3-thienyl)-buttersäure, -3-(2-Thienyl)-serin, (Z)-Dehydrophenylalanin, (E)-Dehydrophenylalanin, 1,3-Dioxolan-2-yl-alanin, N-Pyrrolylalanin und -1-, 3- oder 4-Pyrazolylalanin bedeuten,

sowie deren physiologisch verträgliche Salze, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer, funktioneller Gruppen (eine) temporär eingeführte Schutzgruppe(n) abspaltet und die so erhaltene Verbindung gegebenenfalls in ihr physiologisch verträgliches Salz überführt.

2. Verfahren gemäß Anspruch 1, dadurch gekennzeichnet, daß

$R^1$ wie in Anspruch 1 definiert ist;

$R^2$ Isobutyl, Benzyl oder Cyclohexylmethyl bedeutet;

$R^3$ Wasserstoff, ($C_1$-$C_5$)-Alkyl, ($C_6$-$C_{10}$)-Aryl, ($C_6$-$C_{10}$)-Aryl-($C_1$-$C_4$)-alkyl oder Hydroxy bedeutet;

$R^4$ einen Rest der Formel II bedeutet, worin
$R^5$ für Wasserstoff, $(C_1-C_4)$-Alkyl, $(C_1-C_4)$-Alkoxy, $(C_1-C_4)$-Alkylthio, $(C_1-C_4)$-Alkylamino, Hydroxy, Azido, Fluor, Chlor, Brom oder Jod steht,
D wie in Anspruch 1 definiert ist, und
m 0, 1 oder 2 bedeutet; und
A und B unabhängig voneinander einen bivalenten Rest aus der Reihe
Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, β-3-Furylalanin, Lysin, Ornithin Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, Norleucin, Cystein, S-Methyl-Cystein, 1,2,3,4-Tetrahydroisochinolin-3-carbonsäure, Homophenylalanin, 2-Amino-4-(2-thienyl)-buttersäure, 2-Amino-4-(3-thienyl)-buttersäure, 3-(2-Thienyl)-serin, (Z)-Dehydrophenylalanin, (E)-Dehydrophenylalanin, 1,3-Dioxolan-2-yl-alanin, N-Pyrrolylalanin und 1-, 3- oder 4-Pyrazolylalanin, bedeuten.

3. Verfahren gemäß Anspruch 1 und/oder 2, dadurch gekennzeichnet, daß
$R^1$ Het-$(C_1-C_4)$-alkyl,
Het-$(C_1-C_4)$-alkoxy,
Het-$(C_5-C_6)$-cycloalkyl,
Het-$(C_5-C_6)$-cycloalkoxy,
Het-mercapto-$(C_1-C_3)$-alkyl,
Het-mercapto-$(C_5-C_6)$-cycloalkyl,
Het-mercapto-$(C_1-C_3)$-alkoxy,
Het-mercapto-$(C_5-C_6)$-cycloalkoxy bedeutet,
wobei Het für einen 5- bis 6-gliedrigen heterocyclischen Ring steht, wie z.B. Furanyl, Thienyl, Pyrrolyl, Imidazolyl, Isoxazolyl, Thiazolyl, Pyrazolyl, Triazolyl, Pyridyl, Pyrimidyl, Pyrazinyl, Pyridazinyl, Triazinyl, Pyrrolidyl, Piperidyl, Piperazinyl, Morpholino, Thiomorpholino, Tetrahydrofuranyl, Tetrahydropyranyl, Tetrahydrothienyl, und durch einen oder zwei gleiche oder verschiedene Reste aus der Gruppe $(C_1-C_4)$-Alkyl, $(C_1-C_4)$-Alkoxy, $(C_1-C_4)$-Alkoxycarbonyl, Hydroxy, Amino, Mono- oder Di-$(C_1-C_4)$-Alkylamino und Oxido substituiert sein kann;
$R^2$ Isobutyl, Benzyl oder Cyclohexylmethyl bedeutet,
$R^3$ Wasserstoff oder Hydroxy bedeutet;
$R^4$ einen Rest der Formel II bedeutet, in welcher
$R^5$ für Wasserstoff oder Fluor steht,
D für einen 2-, 3- oder 4-Pyridinrest, einen 2-, 4- oder 5-Imidazolrest oder einen 2-Oxazolinrest steht, wobei die genannten Heterocyclen jeweils durch einen oder zwei Reste aus der Gruppe Methyl, Methoxy, Fluor, Chlor, Brom und $CF_3$ substituiert sein können, und
m 0, 1 oder 2 bedeutet; und
A und B unabhängig voneinander einen bivalenten Rest aus der Reihe
Phenylalanin, Histidin, Tyrosin, Tryptophan, Methionin, Leucin, Isoleucin, Asparagin, Asparaginsäure, β-2-Thienylalanin, β-3-Thienylalanin, β-2-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, Norleucin, 1,2,3,4-Tetrahydroisochinolin-3-carbonsäure, Homophenylalanin, 2-Amino-4-(2-thienyl)-buttersäure und 1-, 3- und 4-Pyrazolylalanin bedeuten.

4. Verwendung einer nach einem der Verfahren der Ansprüche 1 bis 3 hergestellten Verbindung der Formel I als Heilmittel.

5. Verwendung einer nach einem der Verfahren der Ansprüche 1 bis 3 hergestellten Verbindung der Formel I bei der Behandlung des Bluthochdrucks.

6. Verwendung einer nach einem der Verfahren der Ansprüche 1 bis 3 hergestellten Verbindung der Formel I bei der Behandlung viraler Erkrankungen.

7. Verfahren zur Herstellung einer pharmazeutischen Zubereitung enthaltend eine nach einem der Verfahren der Ansprüche 1 bis 3 hergestellten Verbindung der Formel I, dadurch gekennzeichnet, daß man diese zusammen mit einem physiologisch unbedenklichen Träger und gegebenenfalls weiteren Hilfs- und Zusatzstoffen in eine geeignete Darreichungsform bringt.